(19) Europäisches Patentamt
European Patent Office
Office européen des brevets

(11) EP 4 659 737 A1

(12) # EUROPEAN PATENT APPLICATION
published in accordance with Art. 153(4) EPC

(43) Date of publication:
**10.12.2025 Bulletin 2025/50**

(21) Application number: **24750208.1**

(22) Date of filing: **29.01.2024**

(51) International Patent Classification (IPC):
*A61K 8/49* (2006.01) *A61K 8/02* (2006.01)
*A61K 8/19* (2006.01) *A61K 8/20* (2006.01)
*A61K 8/23* (2006.01) *A61K 8/36* (2006.01)
*A61K 8/41* (2006.01) *A61Q 5/06* (2006.01)

(52) Cooperative Patent Classification (CPC):
**A61K 8/02; A61K 8/19; A61K 8/20; A61K 8/23; A61K 8/36; A61K 8/41; A61K 8/49; A61Q 5/06; A61Q 5/10**

(86) International application number:
**PCT/JP2024/002645**

(87) International publication number:
**WO 2024/162259 (08.08.2024 Gazette 2024/32)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.01.2023 JP 2023011707**

(71) Applicant: **Kao Corporation**
**Chuo-ku,**
**Tokyo 103-8210 (JP)**

(72) Inventor: **NAKANO Yujiro**
**Tokyo 131-8501 (JP)**

(74) Representative: **Hoffmann Eitle**
**Patent- und Rechtsanwälte PartmbB**
**Arabellastraße 30**
**81925 München (DE)**

(54) **HAIR DYEING METHOD**

(57) A method for dyeing hair, comprising in order: step 1: applying an A part comprising the following components (a1) and (a2): (a1) a compound of the following formula (1) or a salt thereof: wherein the broken line represents presence or absence of a $\pi$ bond; $R^1$ represents a hydroxy group or an acetoxy group; $R^2$ represents a hydrogen atom or -COOR (wherein R represents a hydrogen atom, a methyl group, or an ethyl group); and $R^3$ represents a hydrogen atom, an acetyl group, a methyl group, or an ethyl group, and (a2) an alkali agent and having pH of from 8 to 11 to hair; and step 2: applying a B part comprising the following component (b): (b) an alkali metal salt or an alkaline earth metal salt selected from the group consisting of an organic acid salt having 6 or less carbon atoms and an inorganic salt to the hair, the method having no step of rinsing hair between the step 1 and the step 2 and further comprising a step of rinsing hair after the step 2.

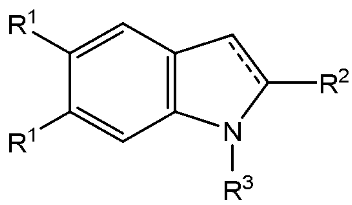 (1)

EP 4 659 737 A1

## Description

Field of the Invention

The present invention relates to a method for dyeing hair.

Background of the Invention

Air oxidation hair dyes employing 5,6-dihydroxyindole, 5,6-dihydroxyindoline, or a derivative thereof, which is a melanin precursor, have heretofore been known as hair dyes for gray hair dyeing. These melanin precursors can dye hair without the use of an oxidizing agent and therefore cause less damage on keratin fiber when used in hair dyes, and are highly convenient as dyes for hair dyes.

For example, JP-A-sho-63-170309 (JP-A-1988-170309, Patent Literature 1) discloses a method for dyeing keratinous fiber with an indole derivative combined with iodide, with an object to dye human keratinous fiber with an indole derivative capable of producing a vivid color, without deteriorating hair, in very short exposure time.

JP-A-hei-1-319413 (JP-A-1989-319413, Patent Literature 2) discloses a method for dyeing hair by continuous treatments with a metal ion-containing composition and 5,6-dihydroxyindole-2-carboxylic acid-containing dye composition, with an object to obtain a dyed product which has natural sleekness and luster, has no coated feeling, and has photostability and shampoo stability as a result of exhibiting retention against light and wetness.

JP-A-hei-8-505155 (JP-A-1996-605155, Patent Literature 3) discloses a method using a simple salt or polymer of inorganic or organic magnesium soluble in a medium in a method for dyeing keratin fiber, particularly, human hair using 5,6-dihydroxyindole and (or) a derivative thereof as a dye, with an object to perform dyeing which exhibits favorable resistance to repetitive washing and sweating while improving durability against light beam.

Summary of the Invention

The present invention relates to the following [1].

[1] A method for dyeing hair comprising in order:

step 1: applying an A part comprising the following components (a1) and (a2):

(a1) a compound of the following formula (1) or a salt thereof:

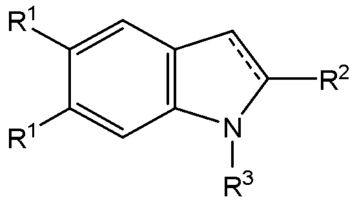

(1)

wherein the broken line represents the presence or absence of a $\pi$ bond; $R^1$ represents a hydroxy group or an acetoxy group; $R^2$ represents a hydrogen atom or -COOR, wherein R represents a hydrogen atom, a methyl group, or an ethyl group; and $R^3$ represents a hydrogen atom, an acetyl group, a methyl group, or an ethyl group, and
(a2) an alkali agent

and having pH of from 8 to 11 to hair; and
step 2: applying a B part comprising the following component (b):
(b) an alkali metal salt or an alkaline earth metal salt selected from the group consisting of an organic acid salt having 6 or less carbon atoms and an inorganic salt to the hair,
the method having no step of rinsing hair between the step 1 and the step 2 and further comprising a step of rinsing hair after the step 2.

Detailed Description of the Invention

However, the method described in Patent Literature 1 exploits the color development of a hair color into brown

color in association with the generation of iodine through the reaction of iodide with hydrogen peroxide, and disadvantageously causes damage on hair because hydrogen peroxide is essential. The method described in Patent Literature 2 treats hair with a transition metal ion-containing composition, which may therefore exhibit toxicity or physiological activity due to its interaction with a biological organic molecule and disadvantageously influences hair dyeing after the treatment. Furthermore, an ion concentration or the like in water differs among regions. When hair to which a melanin precursor has been applied is rinsed with water having a distinctive ion concentration or the like, hair dyeability is disadvantageously influenced thereby, and the method described in Patent Literature 3 had room for improvement in hair dyeability.

The present invention relates to a method for dyeing hair which causes no damage ascribable to oxidation, achieves stable hair dyeing performance of a melanin precursor by a simple process, and can further improve hair dyeability.

The present inventor completed the present invention by finding that the problems described above can be solved by a method for dyeing hair comprising: step 1: applying an A part comprising a certain melanin precursor and an alkali agent and having pH in a specific range to hair; and step 2: applying a B part comprising a specific alkali metal salt or alkaline earth metal salt to the hair, the method having no step of rinsing hair between the step 1 and the step 2 and comprising a step of rinsing hair after the step 2.

The present invention can provide a method for dyeing hair which causes no damage ascribable to oxidation, achieves stable hair dyeing performance of a melanin precursor by a simple process, and can further improve hair dyeability.

[Definition]

In the present specification, the term "favorable hair dyeability" means a high degree of hair dyeing. The high degree of hair dyeing means a large color difference ($\Delta E$) of hair between before and after dyeing, which specifically can be evaluated by a method described in Examples.

In the present specification, the phrase "stable hair dyeing performance of a melanin precursor" means that a process having, after application of the A part, the step of applying the B part to hair such that the B part is applied onto the A part applied to the hair, without rinsing, is insusceptible to an ion concentration or the like of water different among regions and stably exhibits favorable hair dyeability, as compared with a process having no such step.

[Method for dyeing hair]

The method for dyeing hair of the present invention (hereinafter, also simply referred to as the "method of the present invention") includes in order:

step 1: applying an A part containing the following components (a1) and (a2):

(a1) a compound of the following formula (1) or a salt thereof:

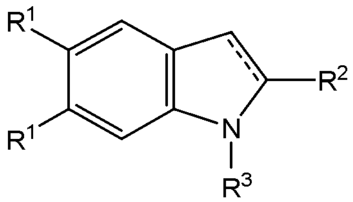

(1)

wherein the broken line represents the presence or absence of a $\pi$ bond; $R^1$ represents a hydroxy group or an acetoxy group; $R^2$ represents a hydrogen atom or -COOR (wherein R represents a hydrogen atom, a methyl group, or an ethyl group); and $R^3$ represents a hydrogen atom, an acetyl group, a methyl group, or an ethyl group, and

(a2) an alkali agent

and having pH of from 8 to 11 to hair; and

step 2: applying a B part containing the following component (b):

(b) an alkali metal salt or an alkaline earth metal salt selected from the group consisting of an organic acid salt having 6 or less carbon atoms and an inorganic salt to the hair,

the method having no step of rinsing hair between the step 1 and the step 2 and further comprising a step of rinsing hair after the step 2.

The present invention, by having the configuration described above, i.e., performing step 1: applying an A part to hair and then step 2: applying a B part to the hair to which the applied A part has been attached, can provide a method for dyeing hair which causes no damage ascribable to oxidation, achieves stable hair dyeing performance of a melanin precursor by a simple process, and can further improve hair dyeability.

The present invention exerts the effects described above, presumably because:
in the method of the present invention, a treatment is performed by applying an A part including a certain melanin precursor and an alkali agent and having pH in a specific range to hair, and then applying a B part containing a specific alkali metal salt or alkaline earth metal salt to the hair so as to coat the applied A part, without washing off the A part. Such a treatment elevates an ion concentration in hair and is therefore insusceptible to an ion concentration or the like of water for rinsing the hair, achieves stable hair dyeing performance of a melanin precursor by a simple process without a complicated operation, and can improve hair dyeability.

<Step 1>

The step 1 involves applying an A part containing the following components (a1) and (a2):

(a1) a compound of the following formula (1) or a salt thereof:

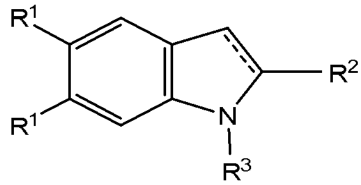

(1)

wherein the broken line represents the presence or absence of a $\pi$ bond; $R^1$ represents a hydroxy group or an acetoxy group; $R^2$ represents a hydrogen atom or -COOR (wherein R represents a hydrogen atom, a methyl group, or an ethyl group); and $R^3$ represents a hydrogen atom, an acetyl group, a methyl group, or an ethyl group, and
(a2) an alkali agent

and having pH of from 8 to 11 to hair.

(A part)

The A part for use in the step 1 contains the following components (a1) and (a2):

(a1) a compound of the following formula (1) or a salt thereof:

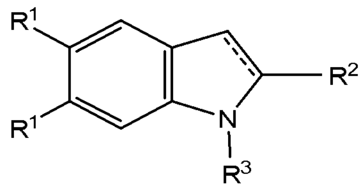

(1)

wherein the broken line represents the presence or absence of a $\pi$ bond; $R^1$ represents a hydroxy group or an acetoxy group; $R^2$ represents a hydrogen atom or -COOR (wherein R represents a hydrogen atom, a methyl group, or an ethyl group); and $R^3$ represents a hydrogen atom, an acetyl group, a methyl group, or an ethyl group, and
(a2) an alkali agent

and has pH of from 8 to 11.

<<Component (a1): melanin precursor>>

The A part contains a component (a1) which is a compound of the formula (1) or a salt thereof. The component (a1) is a melanin precursor which is polymerized by air oxidation and thereby converted to a melanin pigment, and acts as a hair dye.

The compound of the formula (1) is an indole derivative, an indoline derivative, or a salt thereof. In the present

invention, one or more thereof can be used (in combination).

The component (a1) is more preferably an indole derivative (i.e., a π bond is present in a moiety of the broken line in the formula (1)) from the viewpoint of improvement in hair dyeability.

In the formula (1), $R^1$ is preferably a hydroxy group, and $R^2$ is preferably a hydrogen atom or -COOR (wherein R is a hydrogen atom, a methyl group, or an ethyl group), more preferably a hydrogen atom or -COOH, from the viewpoint of availability of the component (a1) and improvement in hair dyeability. $R^3$ is preferably a hydrogen atom.

Examples of the compound of the formula (1) include 5,6-dihydroxyindole, 5,6-dihydroxyindole-2-carboxylic acid, methyl 5,6-dihydroxyindole-2-carboxylate, ethyl 5,6-dihydroxyindole-2-carboxylate, N-methyl-5,6-dihydroxyindole, N-methyl-5,6-dihydroxyindole-2-carboxylic acid, N-ethyl-5,6-dihydroxyindole, N-ethyl-5,6-dihydroxyindole-2-carboxylic acid, N-acetyl-5,6-dihydroxyindole, N-acetyl-5,6-dihydroxyindole-2-carboxylic acid, 5-acetoxy-6-hydroxyindole, 5-acetoxy-6-hydroxyindole-2-carboxylic acid, 5,6-dihydroxyindoline, 5,6-dihydroxyindoline-2-carboxylic acid, methyl 5,6-dihydroxyindoline-2-carboxylate, ethyl 5,6-dihydroxyindoline-2-carboxylate, N-methyl-5,6-dihydroxyindoline, N-methyl-5,6-dihydroxyindoline-2-carboxylic acid, N-ethyl-5,6-dihydroxyindoline, N-ethyl-5,6-dihydroxyindoline-2-carboxylic acid, N-acetyl-5,6-dihydroxyindoline, N-acetyl-5,6-dihydroxyindoline-2-carboxylic acid, 5-acetoxy-6-hydroxyindoline, and 5-acetoxy-6-hydroxyindoline-2-carboxylic acid, one or more of which can be used.

Examples of the salt of the compound of the formula (1) include hydrochloride, hydrobromide, sulfate, phosphate, acetate, propionate, lactate, and citrate of any of the compounds. Hydrobromide is preferred from the viewpoint of availability and improvement in hair dyeability.

When $R^2$ in the formula (1) is -COOH, examples of the salt of the compound of the formula (1) include carboxylate thereof (wherein $R^2$ is $-COO^-X^+$ ($X^+$ is a cation such as an alkali metal ion (e.g., $Na^+$ and $K^+$), an alkaline earth metal ion (e.g., $Ca^+$ and $Mg^+$), or an ammonium ion)).

The component (a1) preferably contains one or more members selected from the group consisting of 5,6-dihydroxyindole, 5,6-dihydroxyindole-2-carboxylic acid, 5,6-dihydroxyindoline, 5,6-dihydroxyindoline-2-carboxylic acid, and a salt thereof, more preferably one or more members selected from the group consisting of 5,6-dihydroxyindole, 5,6-dihydroxyindole-2-carboxylic acid, and 5,6-dihydroxyindoline hydrobromide, further more preferably one or more members selected from the group consisting of 5,6-dihydroxyindole and 5,6-dihydroxyindole-2-carboxylic acid, and even more preferably 5,6-dihydroxyindole and 5,6-dihydroxyindole-2-carboxylic acid, from the viewpoint of improvement in hair dyeability.

When the component (a1) contains 5,6-dihydroxyindole and 5,6-dihydroxyindole-2-carboxylic acid, a molar ratio of the 5,6-dihydroxyindole and the 5,6-dihydroxyindole-2-carboxylic acid is preferably from 50 : 50 to 99 : 1, more preferably from 80 : 20 to 99 : 1, further more preferably from 85 : 15 to 95 : 5, from the viewpoint of improvement in hair dyeability.

When the component (a1) contains 5,6-dihydroxyindole and 5,6-dihydroxyindole-2-carboxylic acid, the molar ratio of the 5,6-dihydroxyindole and the 5,6-dihydroxyindole-2-carboxylic acid in the component (a1) can be quantified by reverse-phase HPLC.

A content of the component (a1) in the A part is preferably 0.02% by mass or more, more preferably 0.05% by mass or more, further more preferably 0.1% by mass or more, from the viewpoint of improvement in hair dyeability, and preferably 5.0% by mass or less, more preferably 3.0% by mass or less, further more preferably 2.0% by mass or less, even more preferably 1.0% by mass or less, even more preferably 0.8% by mass or less, even more preferably 0.6% by mass or less, even more preferably 0.5% by mass or less, from the viewpoint of economic performance. The content of the component (a1) in the A part is preferably from 0.02 to 5.0% by mass, more preferably from 0.05 to 3.0% by mass, further more preferably from 0.05 to 2.0% by mass, even more preferably from 0.05 to 1.0% by mass, even more preferably from 0.1 to 0.8% by mass, even more preferably from 0.1 to 0.6% by mass, even more preferably from 0.1 to 0.5% by mass, from the viewpoint described above.

<<Component (a2): alkali agent>>

The component (a2) exerts an effect of improving hair dyeability through the action of swelling hair so as to open cuticle and allowing a coloring component such as the component (a1) to penetrate the inside of the hair.

Any alkali agent for use in a conventional hair dye composition can be used without particular limitations as the component (a2), which may be any of inorganic alkali agents and organic alkali agents. The component (a2) is preferably ammonia or an organic alkali agent from the viewpoint of improvement in hair dyeability.

Examples of the alkali agent which is preferred as the component (a2) include: ammonia; alkanolamine such as mono-, di-, or trimethanolamine, mono-, di-, or triethanolamine, trishydroxymethylaminomethane, and 2-amino-2-methyl-1-propanol; alkylamine such as methylamine, dimethylamine, ethylamine, diethylamine, N-methylethylamine, propylamine, and butylamine; and aralkylamine such as benzylamine, one or more of which can be used. The alkanolamine, the alkylamine, or the aralkylamine has preferably 1 or more and 10 or less, more preferably 1 or more and 8 or less carbon atoms from the viewpoint of water solubility.

Among those described above, the component (a2) preferably contains one or more members selected from the group consisting of ammonia, alkanolamine, alkylamine, and aralkylamine, more preferably contains one or more members selected from the group consisting of ammonia and alkanolamine, and further more preferably contains alkanolamine, from the viewpoint of improvement in hair dyeability. The component (a2) preferably contains one or more members selected from the group consisting of monoethanolamine, trishydroxymethylaminomethane, and 2-amino-2-methyl-1-propanol, more preferably contains one or more members selected from the group consisting of monoethanolamine and 2-amino-2-methyl-1-propanol, and further more preferably contains monoethanolamine, as the alkanolamine from the viewpoint of improvement in hair dyeability.

A content of the alkanolamine in the component (a2) is preferably 70% by mass or more, more preferably 80% by mass or more, further more preferably 90% by mass or more, and 100% by mass or less, from the viewpoint of improvement in hair dyeability.

A content of the component (a2) in the A part is preferably 0.01% by mass or more, more preferably 0.1% by mass or more, further more preferably 0.5% by mass or more, even more preferably 1.0% by mass or more, even more preferably 2.0% by mass or more, from the viewpoint of allowing the component (a1) to penetrate the inside of hair while improving hair dyeability by accelerating the polymerization reaction of the component (a1) in the hair, and preferably 10% by mass or less, more preferably 7.5% by mass or less, further more preferably 5.0% by mass or less, from the viewpoint of suppressing hair damage. The content of the component (a2) in the A part is preferably from 0.01 to 10% by mass, more preferably from 0.1 to 7.5% by mass, further more preferably from 0.5 to 5.0% by mass, even more preferably from 1.0 to 5.0% by mass, even more preferably from 2.0 to 5.0% by mass, from the viewpoint described above.

<<pH adjuster>>

The A part preferably further contains a pH adjuster from the viewpoint of adjusting pH to a desired range mentioned later, accelerating the polymerization reaction within hair of the component (a1), and improving hair dyeability.

Since the A part contains the alkali agent as the component (a2), the pH adjuster for use in the A part is preferably a protonating agent. The protonating agent may be any of monobasic acids and polybasic acids or may be any of organic acids (having 1 or more and 8 or less carbon atoms, except for ascorbic acid) and inorganic acids.

Examples of the protonating agent include one or more members selected from the group consisting of hydrochloric acid, sulfuric acid, phosphoric acid, formic acid, acetic acid, and citric acid. One or more members selected from the group consisting of phosphoric acid and citric acid is preferred from the viewpoint of adjusting pH of the A part to a range mentioned later.

When the A part contains the pH adjuster, a content of the pH adjuster in the A part is preferably 0.05% by mass or more, more preferably 0.1% by mass or more, further more preferably 0.2% by mass or more, and preferably 5.0% by mass or less, more preferably 4.0% by mass or less, further more preferably 3.5% by mass or less, even more preferably 2.5% by mass or less, even more preferably 1.5% by mass or less, from the viewpoint of adjusting pH of the A part to a range mentioned later.

<<Other components>>

The A part may appropriately contain, in addition to the components described above, components which are conventionally used in hair dye compositions, without impairing the object of the present invention. Examples of the components include aqueous media such as water, lower alcohols, and low-molecular diol and triol having 6 or less carbon atoms, surfactants, viscosity adjusters, silicone, aromatic alcohols, coloring components other than the component (a1), polymers, oil agents, anti-dandruff agents, vitamin preparations, germicides, anti-inflammatory agents, antiseptics, chelating agents, humectants, pearlescent agents, a ceramide, fragrances, and ultraviolet absorbers.

The A part may contain the component (b) mentioned later, and a content of the component (B) in the A part is preferably 10% by mass or less, more preferably 8% by mass or less, more preferably 5% by mass or less, further more preferably 3.5% by mass or less, from the viewpoint of hair dyeability.

<<pH>>

The pH of the A part is 8 or higher, preferably 9 or higher, more preferably 9.5 or higher, further more preferably 9.8 or higher, from the viewpoint of improving hair dyeability. The pH is 11 or lower, preferably 10.8 or lower, more preferably 10.5 or lower from the viewpoint of improving hair dyeability and from the viewpoint of suppressing hair damage. The pH of the A part is from 8 to 11, preferably from 9 to 10.8, more preferably from 9.5 to 10.8, further more preferably from 9.8 to 10.5, from the viewpoint described above.

The pH is a value measured at 25°C and, specifically, can be measured in accordance with a method described in Examples.

Preferably, the A part substantially contains no oxidizing agent (e.g., hydrogen peroxide or sodium bromate) from the viewpoint of suppressing hair damage. The phrase "A part substantially contains no oxidizing agent" means that a content of the oxidizing agent in the A part is preferably less than 0.1% by mass, more preferably less than 0.01% by mass, further more preferably 0% by mass.
A formulation of the A part is not particularly limited and the A part may be prepared in any formulation, for example, a liquid, a foam, a paste, a cream, a solid, or a powder. The formulation of the A part is preferably a liquid, a paste, or a cream from the viewpoint of application to hair by coating, immersion, or the like.
A method for preparing the A part is not particularly limited. This part can be prepared, for example, by blending the component (a1), the component (a2), and other optional components and mixing these components with a known stirring apparatus or the like. The A part is preferably prepared in an inert gas (e.g., nitrogen gas) atmosphere from the viewpoint of avoiding polymerization of the component (a1).

(Application of A part to hair)

In the step 1, the hair in applying the A part thereto may be in a dry state or a wet state. A method for applying the A part to hair may not be limited as long as the method facilitates to contact the A part with the hair. Examples thereof include a method of coating the hair with the A part and a method of immersing the hair in the A part.
In the step 1, an amount of the A part applied to hair is a bath ratio (dry mass of the hair : mass of the A part) of preferably from 1 : 0.05 to 1 : 20, more preferably from 1 : 0.3 to 1 : 10, further more preferably from 1 : 0.5 to 1 : 5, from the viewpoint of balance between hair dyeability and economic performance. The dry mass of the hair means a mass of hair cleansed with a shampoo, blow-dried with hot air of a dryer for 30 minutes, and left to stand overnight or longer at temperature of 25°C under a relative humidity of 65%.
The hair to which the A part is to be applied is preferably head hair and can be at least a portion of the head hair.
In applying the A part to hair by coating the hair with the A part in the step 1, coating time is preferably 10 seconds or longer, more preferably 30 seconds or longer, further more preferably 1 minute or longer, from the viewpoint of allowing the components of the A part to penetrate the inside of the hair, and preferably 10 minutes or shorter, more preferably 5 minutes or shorter, from the viewpoint of reducing burden on a practitioner.
In applying the A part to hair by immersing the hair in the A part in the step 1, immersion time is preferably 1 minute or longer, more preferably 3 minutes or longer, and preferably 30 minutes or shorter, more preferably 20 minutes or shorter, from the viewpoint described above.
In the step 1, after the A part is applied to hair by coating, immersion, or the like, the resultant is preferably further left to stand. Total time for which the resultant is left to stand can be appropriately changed in accordance with a desired color of hair and is preferably 1 minute or longer, more preferably 3 minutes or longer, further more preferably 5 minutes or longer, from the viewpoint of allowing the components of the A part to penetrate the inside of the hair, and preferably 60 minutes or shorter, more preferably 30 minutes or shorter, further more preferably 20 minutes or shorter, from the viewpoint of reducing burden on a practitioner. The time for which the A part is applied to hair is preferably from 1 to 60 minutes, more preferably from 3 to 30 minutes, further more preferably from 5 to 20 minutes, from the viewpoint described above.
In the step 1, temperature in applying the A part to hair is not particularly limited and is preferably 40°C or lower, more preferably 35°C or lower, from the viewpoint of the handleability of the A part, and preferably 15°C or higher, more preferably 20°C or higher, further more preferably 25°C or higher, from the viewpoint of improvement in hair dyeability. The temperature in applying the A part to hair is preferably from 15 to 40°C, more preferably from 20 to 35°C, from the viewpoint described above. When the A part is left to stand after being applied, temperature in leaving the A part to stand is not particularly limited and is preferably from 25 to 50°C, more preferably from 30 to 40°C.
The method of the present invention has no step of rinsing hair (hereinafter, also simply referred to as a "rinsing step") after applying the A part to hair and before applying the B part to the hair, from the viewpoint of achieving stable hair dyeing performance of a melanin precursor and improving hair dyeability. Specifically, the B part is applied to the hair with an A part being applied.

(B part)

The B part for use in the step 2 contains the following component (b):
(b) an alkali metal salt or an alkaline earth metal salt selected from the group consisting of an organic acid salt having 6 or less carbon atoms and an inorganic salt.

<<Component (b)>>

The component (b) exerts an effect of elevating an ion concentration so that hair is insusceptible to an ion concentration of rinsing water or the like. Examples of the alkali metal salt include sodium salt and potassium salt.

Examples of the alkaline earth metal salt include calcium salt and magnesium salt. One or more of these salts can be used. Examples of the organic acid salt having 6 or less carbon atoms include carboxylate, dicarboxylate, citrate, malate, succinate, and lactate. Examples of the inorganic salt include nitrate, sulfate, and chloride.

Among them, the component (b) is preferably one or more members selected from the group consisting of chloride, carboxylate, sulfate, and carbonate of sodium, potassium, calcium, or magnesium, more preferably one or more members selected from the group consisting of chloride, carboxylate, and sulfate of sodium, potassium, calcium, or magnesium, further more preferably one or more members selected from the group consisting of sodium chloride, potassium chloride, calcium chloride, magnesium chloride, calcium acetate, potassium acetate, calcium sulfate, and potassium sulfate, even more preferably one or more members selected from the group consisting of sodium chloride, magnesium chloride, calcium acetate, and potassium sulfate, from the viewpoint of more stabilizing the hair dyeing performance of a melanin precursor and improving hair dyeability.

A content of the component (b) in the B part is preferably 0.001% by mass or more, more preferably 0.005% by mass or more, more preferably 0.01% by mass or more, from the viewpoint of more stabilizing the hair dyeing performance of a melanin precursor and improving hair dyeability, and preferably 95% by mass or less, more preferably 85% by mass or less, further more preferably 80% by mass or less, from the viewpoint of economic performance and ease of application to hair. The content of the component (b) in the B part is preferably from 0.001 to 95% by mass, more preferably from 0.005 to 85% by mass, further more preferably from 0.01 to 80% by mass, from the viewpoint described above.

The content of the component (b) in the B part is preferably 0.05 to 30% by mass, even more preferably 0.1 to 10% by mass, when the B part is in a liquid or a foam, and is preferably 50 to 95% by mass, more preferably 60 to 80% by mass, when the B part is in a paste or a cream.

<<Other components>>

The B part may appropriately comprise, in addition to the components described above, components which are conventionally used in hair dye compositions, without impairing the object of the present invention. Examples of the components include aqueous media such as water, lower alcohols, and low-molecular diol and triol having 6 or less carbon atoms, surfactants, viscosity adjusters, silicone, aromatic alcohols, polymers, oil agents, anti-dandruff agents, vitamin preparations, germicides, anti-inflammatory agents, antiseptics, chelating agents, humectants, pearlescent agents, a ceramide, fragrances, and ultraviolet absorbers.

Preferably, the B part substantially contains no oxidizing agent from the viewpoint of suppressing hair damage. The phrase "B part substantially contains no oxidizing agent" means that a content of the oxidizing agent in the B part is preferably less than 0.1% by mass, more preferably less than 0.01% by mass, further more preferably 0% by mass. Specifically, in the method for dyeing hair of the present invention, preferably, neither the A part nor the B part contains an oxidizing agent. Examples of the oxidizing agent include those listed in the A part mentioned above. The content of the oxidizing agent also means the content described in the A part.

A formulation of the B part is not particularly limited and may be any formulation, for example, a liquid, a foam, a paste, a cream, a solid, or a powder. The formulation is preferably a liquid, a foam, a paste, or a cream.

A method for preparing the B part is not particularly limited. This part can be prepared, for example, by blending the component (b) and other optional components and mixing these components with a known stirring apparatus or the like.

(Application of B part to hair)

In the step 2, the hair in applying the B part thereto is the hair to which the A part has been applied. A method for applying the B part to the hair may not be limited as long as the method can contact the B part with the hair. Examples thereof include a method of coating the hair with the B part and a method of immersing the hair in the B part. Among them, a method of coating the hair with the B part is preferred from the viewpoint of more stabilizing the hair dyeing performance of a melanin precursor and improving hair dyeability.

In the step 2, the B part is preferably applied to the hair at a bath ratio (dry mass of the hair : mass of the component (b)) of 1 : 0.00005 or more, preferably applied to the hair at a bath ratio of from 1 : 0.00005 to 1 : 1, more preferably applied to the hair at a bath ratio of from 1 : 0.005 to 1 : 0.5, and further more preferably applied to the hair at a bath ratio of from 1 : 0.012 to 1 : 0.15, from the viewpoint of more stabilizing the hair dyeing performance of a melanin precursor and improving hair dyeability and from the viewpoint of economic performance.

In applying the B part to the hair by coating the hair with the B part in the step 2, coating time is preferably 10 seconds or longer, more preferably 30 seconds or longer, further more preferably 1 minute or longer, from the viewpoint of more stabilizing the hair dyeing performance of a melanin precursor and improving hair dyeability, and preferably 10 minutes or shorter, more preferably 5 minutes or shorter, from the viewpoint of reducing burden on a practitioner.

After the step 2 of applying the B part to the hair, a step of spreading the B part throughout the hair in preferably

further performed. Examples of a method for spreading the B part throughout the hair include a method of rubbing the B part applied to the hair onto the hair. In this respect, the spreading time of the B part throughout the hair is preferably 20 seconds or longer, more preferably 40 seconds or longer, further more preferably 50 seconds or longer, from the viewpoint of more stabilizing the hair dyeing performance of a melanin precursor and improving hair dyeability, and preferably 20 minutes or shorter, more preferably 10 minutes or shorter, further more preferably 3 minutes or shorter, from the viewpoint of reducing burden on a practitioner. The spreading time after the application of the B part to the hair is preferably from 20 seconds to 20 minutes, more preferably from 40 seconds to 10 minutes, further more preferably from 50 seconds to 3 minutes, from the viewpoint described above.

In the step 2, temperature in applying the B part to the hair is not particularly limited and is preferably 40°C or lower, more preferably 35°C or lower, from the viewpoint of the handleability of the B part, and preferably 15°C or higher, more preferably 20°C or higher, from the viewpoint of improvement in hair dyeability. The temperature in applying the B part to the hair is preferably from 15 to 40°C, more preferably 20 to 35°C, from the viewpoint described above.

<Rinsing step>

The method of the present invention includes, after the step 2, a step of rinsing the A part and the B part applied to the hair (hereinafter, also simply referred to as a "rinsing step"). The rinsing step is performed, for example, by washing off the A part and the B part applied to the hair with water. Temperature of the water is not particularly limited, and water of 35 to 45°C is preferred. Rinsing time of the hair is not particularly limited and is preferably 10 seconds or longer, more preferably 20 seconds or longer, and preferably 5 minutes or shorter, more preferably 3 minutes or shorter, further more preferably 1 minute or shorter, from the viewpoint of more stabilizing the hair dyeing performance of a melanin precursor and improving hair dyeability.

<Drying step>

The method of the present invention preferably includes, after the rinsing step, a step of drying the hair (hereinafter, also simply referred to as a "drying step"). The drying step is a step of reducing an amount of water in the hair and includes, for example, performing one or more drying treatments selected from the group consisting of towel drying, blow (cold air or hot air) drying, and air drying in order to actively reduce the amount of water in the hair. Two or more of these drying treatments are preferably performed in combination.

Time from the completion of the rinsing step to the start of the drying step is preferably 20 minutes or shorter, more preferably 17 minutes or shorter, further more preferably 15 minutes or shorter, from the viewpoint of more stabilizing the hair dyeing performance of a melanin precursor and improving hair dyeability, and a lower limit thereof is not particularly limited and is 10 seconds or longer.

Hereinafter, in relation to the embodiments mentioned above, the present invention further discloses the following.

<1> A method for dyeing hair comprising in order:

step 1: applying an A part comprising the following components (a1) and (a2):

(a1) a compound of the following formula (1) or a salt thereof:

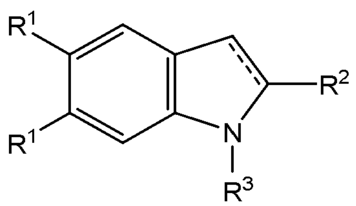

(1)

wherein the broken line represents the presence or absence of a $\pi$ bond; $R^1$ represents a hydroxy group or an acetoxy group; $R^2$ represents a hydrogen atom or -COOR (wherein R represents a hydrogen atom, a methyl group, or an ethyl group); and $R^3$ represents a hydrogen atom, an acetyl group, a methyl group, or an ethyl group, and

(a2) an alkali agent

and having pH of from 8 to 11 to hair; and

step 2: applying a B part comprising the following component (b):
(b) an alkali metal salt or an alkaline earth metal salt selected from the group consisting of an organic acid salt having 6 or less carbon atoms and an inorganic salt to the hair, wherein
the step 2 is performed onto the A part applied to the hair after the step 1, and the method further comprises a step of rinsing hair after the step 2.

<2> The method for dyeing hair according to <1>, wherein the component (a1) is one or more members selected from the group consisting of 5,6-dihydroxyindole, 5,6-dihydroxyindole-2-carboxylic acid, methyl 5,6-dihydroxyindole-2-carboxylate, ethyl 5,6-dihydroxyindole-2-carboxylate, N-methyl-5,6-dihydroxyindole, N-methyl-5,6-dihydroxyindole-2-carboxylic acid, N-ethyl-5,6-dihydroxyindole, N-ethyl-5,6-dihydroxyindole-2-carboxylic acid, N-acetyl-5,6-dihydroxyindole, N-acetyl-5,6-dihydroxyindole-2-carboxylic acid, 5-acetoxy-6-hydroxyindole, 5-acetoxy-6-hydroxyindole-2-carboxylic acid, 5,6-dihydroxyindoline, 5,6-dihydroxyindoline-2-carboxylic acid, methyl 5,6-dihydroxyindoline-2-carboxylate, ethyl 5,6-dihydroxyindoline-2-carboxylate, N-methyl-5,6-dihydroxyindoline, N-methyl-5,6-dihydroxyindoline-2-carboxylic acid, N-ethyl-5,6-dihydroxyindoline, N-ethyl-5,6-dihydroxyindoline-2-carboxylic acid, N-acetyl-5,6-dihydroxyindoline, N-acetyl-5,6-dihydroxyindoline-2-carboxylic acid, 5-acetoxy-6-hydroxyindoline and 5-acetoxy-6-hydroxyindoline-2-carboxylic acid, and hydrochloride, hydrobromide, sulfate, phosphate, acetate, propionate, lactate, and citrate of each of these compounds, preferably hydrobromide of each of these compounds.
<3> The method for dyeing hair according to <1> or <2>, wherein the component (a1) preferably comprises one or more members selected from the group consisting of 5,6-dihydroxyindole, 5,6-dihydroxyindole-2-carboxylic acid, 5,6-dihydroxyindoline, 5,6-dihydroxyindoline-2-carboxylic acid, and a salt thereof, more preferably comprises one or more members selected from the group consisting of 5,6-dihydroxyindole, 5,6-dihydroxyindole-2-carboxylic acid, and 5,6-dihydroxyindoline hydrobromide, further more preferably comprises one or more members selected from the group consisting of 5,6-dihydroxyindole and 5,6-dihydroxyindole-2-carboxylic acid, and even more preferably comprises 5,6-dihydroxyindole and 5,6-dihydroxyindole-2-carboxylic acid mixture.
<4> The method for dyeing hair according to any one of <1> to <3>, wherein when $R^2$ in the formula (1) of the component (a1) is -COOH, the salt of the compound of the formula (1) is carboxylate thereof (wherein $R^2$ is $-COO^-X^+$ ($X^+$ is a cation)).
<5> The method for dyeing hair according to any one of <1> to <4>, wherein when the component (a1) comprises 5,6-dihydroxyindole and 5,6-dihydroxyindole-2-carboxylic acid, a molar ratio of the 5,6-dihydroxyindole and the 5,6-dihydroxyindole-2-carboxylic acid is preferably from 50 : 50 to 99 : 1, more preferably from 80 : 20 to 99 : 1, further more preferably from 85 : 15 to 95 : 5.
<6> The method for dyeing hair according to any one of <1> to <5>, wherein a content of the component (a1) is preferably 0.02 to 5.0% by mass, more preferably 0.05 to 3.0% by mass, further more preferably 0.1 to 2.0% by mass, even more preferably 0.1 to 1.0% by mass, even more preferably 0.1 to 0.8% by mass, even more preferably 0.1 to 0.6% by mass, even more preferably 0.1 to 0.5% by mass.
<7> The method for dyeing hair according to any one of <1> to <6>, wherein the component (a2) is an inorganic alkali agent or an organic alkali agent, preferably ammonia or an organic alkali agent.
<8> The method for dyeing hair according to any one of <1> to <7>, wherein the component (a2) is one or more members selected from the group consisting of ammonia, alkanolamine, alkylamine, and aralkylamine, and the alkanolamine, the alkylamine, or the aralkylamine has preferably 1 or more and 10 or less, more preferably 1 or more and 8 or less carbon atoms.
<9> The method for dyeing hair according to any one of <1> to <8>, wherein the component (a2) preferably comprises one or more members selected from the group consisting of ammonia, alkanolamine, alkylamine, and aralkylamine, more preferably comprises one or more members selected from the group consisting of ammonia and alkanolamine, and further more preferably comprises alkanolamine.
<10> The method for dyeing hair according to any one of <1> to <9>, wherein the component (a2) preferably comprises one or more members selected from the group consisting of monoethanolamine, trishydroxymethylaminomethane, and 2-amino-2-methyl-1-propanol, more preferably comprises one or more members selected from the group consisting of monoethanolamine and 2-amino-2-methyl-1-propanol, and further more preferably comprises monoethanolamine.
<11> The method for dyeing hair according to any one of <1> to <10>, wherein a content of the alkanolamine in the component (a2) is preferably 70% by mass or more, more preferably 80% by mass or more, further more preferably 90% by mass or more, and 100% by mass or less.
<12> The method for dyeing hair according to any one of <1> to <11>, wherein a content of the component (a2) in the A part is preferably 0.01 to 10% by mass, more preferably 0.1 to 7.5% by mass, further more preferably 0.5 to 5.0% by mass, even more preferably 1.0 to 5.0% by mass, even more preferably 2.0 to 5.0% by mass.
<13> The method for dyeing hair according to any one of <1> to <12>, wherein the A part further comprises a pH adjuster, and the pH adjuster is preferably one or more protonating agents selected from the group consisting of a

monobasic acid and a polybasic acid, or one or more protonating agents selected from the group consisting of an organic acid (having 1 or more and 8 or less carbon atoms, except for ascorbic acid) and an inorganic acid, preferably one or more members selected from the group consisting of hydrochloric acid, sulfuric acid, phosphoric acid, formic acid, acetic acid, and citric acid as the protonating agent, more preferably one or more members selected from the group consisting of phosphoric acid and citric acid.

<14> The method for dyeing hair according to <13>, wherein when the A part comprises the pH adjuster, a content of the pH adjuster in the A part is preferably 0.05% by mass or more, more preferably 0.1% by mass or more, further more preferably 0.2% by mass or more, and preferably 5.0% by mass or less, more preferably 4.0% by mass or less, further more preferably 3.5% by mass or less, even more preferably 2.5% by mass or less, even more preferably 1.5% by mass or less.

<15> The method for dyeing hair according to any one of <1> to <14>, wherein the A part further comprises one or more members selected from an aqueous medium, a surfactant, a viscosity adjuster, silicone, an aromatic alcohol, a coloring component other than a component (a1), a polymer, an oil agent, an anti-dandruff agent, a vitamin preparation, a germicide, an anti-inflammatory agent, an antiseptic, a chelating agent, a humectant, a pearlescent agent, a ceramide, a fragrance, and an ultraviolet absorber.

<16> The method for dyeing hair according to any one of <1> to <15>, wherein pH at 25°C of the A part is from 8 to 11, preferably from 9 to 10.8, more preferably from 9.5 to 10.8, further more preferably from 9.8 to 10.5.

<17> The method for dyeing hair according to any one of <1> to <16>, wherein the A part substantially contains no oxidizing agent.

<18> The method for dyeing hair according to any one of <1> to <17>, wherein a formulation of the A part is a liquid, a foam, a paste, a cream, a solid, or a powder, preferably a liquid, a paste, or a cream.

<19> The method for dyeing hair according to any one of <1> to <18>, wherein the hair in applying the A part thereto is in a dry state or a wet state.

<20> The method for dyeing hair according to any one of <1> to <19>, wherein a method for applying the A part to hair is a method of contacting the A part with the hair, preferably a method of coating the hair with the A part or a method of immersing the hair in the A part.

<21> The method for dyeing hair according to any one of <1> to <20>, wherein an amount of the A part applied to hair is a bath ratio (dry mass of the hair : mass of the A part) of preferably from 1:0.05 to 1:20, more preferably from 1 : 0.3 to 1 : 10, further more preferably from 1 : 0.5 to 1 : 5.

<22> The method for dyeing hair according to any one of <1> to <21>, wherein in applying the A part to hair by coating, coating time is preferably 10 seconds or longer, more preferably 30 seconds or longer, further more preferably 1 minute or longer, and preferably 10 minutes or shorter, more preferably 5 minutes or shorter.

<23> The method for dyeing hair according to any one of <1> to <21>, wherein in applying the A part to hair by immersion, immersion time is preferably 1 minute or longer, more preferably 3 minutes or longer, and preferably 30 minutes or shorter, more preferably 20 minutes or shorter.

<24> The method for dyeing hair according to any one of <1> to <23>, wherein after the A part is applied to hair, the resultant is further left to stand, and total time for which the resultant is left to stand is preferably from 1 to 60 minutes, more preferably from 3 to 30 minutes, further more preferably from 5 to 20 minutes.

<25> The method for dyeing hair according to any one of <1> to <24>, wherein temperature in applying the A part to hair is preferably from 15 to 40°C, more preferably from 20 to 35°C.

<26> The method for dyeing hair according to any one of <1> to <25>, wherein the component (b) comprises one or more members selected from the group consisting of an alkali metal salt and an alkaline earth metal salt.

<27> The method for dyeing hair according to any one of <1> to <26>, wherein the component (b) comprises an organic acid salt having 6 or less carbon atoms comprising one or more members selected from the group consisting of carboxylate, dicarboxylate, citrate, malate, succinate, and lactate.

<28> The method for dyeing hair according to any one of <1> to <27>, wherein the component (b) comprises an inorganic salt comprising one or more members selected from the group consisting of nitrate, sulfate, and chloride.

<29> The method for dyeing hair according to any one of <1> to <28>, wherein the component (b) is one or more members selected from the group consisting of chloride, carboxylate, sulfate, and carbonate of sodium, potassium, calcium, or magnesium.

<30> The method for dyeing hair according to any one of <1> to <29>, wherein a content of the component (b) in the B part is preferably from 0.001 to 95% by mass, more preferably from 0.005 to 85% by mass, further more preferably from 0.01 to 80% by mass.

<31> The method for dyeing hair according to any one of <1> to <30>, wherein the B part further comprises one or more members selected from the group consisting of an aqueous medium, a surfactant, a viscosity adjuster, silicone, an aromatic alcohol, a polymer, an oil agent, an anti-dandruff agent, a vitamin preparation, a sterilizer, an anti-inflammatory agent, an antiseptic, a chelating agent, a humectant, a pearlescent agent, a ceramide, a fragrance, and an ultraviolet absorber.

<32> The method for dyeing hair according to any one of <1> to <31>, wherein the B part substantially contains no oxidizing agent.
<33> The method for dyeing hair according to any one of <1> to <32>, wherein neither the A part nor the B part contains an oxidizing agent.
<34> The method for dyeing hair according to any one of <1> to <33>, wherein the B part is in a formulation of a liquid, a foam, a paste, a cream, a solid, or a powder.
<35> The method for dyeing hair according to any one of <1> to <34>, wherein a method for applying the B part to the hair is a method of contacting the B part with the hair, preferably a method of coating the hair with the B part or a method of immersing the hair in the B part.
<36> The method for dyeing hair according to any one of <1> to <35>, wherein an amount of the B part applied to the hair is a bath ratio (dry mass of the hair : mass of the component (b)) of preferably from 1 : 0.0001 to 1 : 1, more preferably from 1 : 0.001 to 1 : 0.5, further more preferably from 1 : 0.01 to 1 : 0.15.
<37> The method for dyeing hair according to any one of <1> to <36>, wherein in applying the B part to the hair by coating, coating time is preferably 10 seconds or longer, more preferably 30 seconds or longer, further more preferably 1 minute or longer, and preferably 10 minutes or shorter, more preferably 5 minutes or shorter.
<38> The method for dyeing hair according to any one of <1> to <37>, further comprising, after applying the B part to the hair, the step of spreading the B part throughout the hair, wherein spreading time is preferably from 20 seconds to 20 minutes, more preferably from 40 seconds to 10 minutes, further more preferably from 50 seconds to 3 minutes.
<39> The method for dyeing hair according to any one of <1> to <38>, wherein temperature in applying the B part to the hair is preferably from 25 to 40°C, more preferably from 30 to 35°C.
<40> The method for dyeing hair according to any one of <1> to <39>, wherein the rinsing step is performed by washing off the A part and the B part with water, and preferably temperature of the water is from 35 to 45°C.
<41> The method for dyeing hair according to any one of <1> to <40>, wherein rinsing time in the step of rinsing hair is preferably 10 seconds or longer, more preferably 20 seconds or longer, and preferably 5 minutes or shorter, more preferably 3 minutes or shorter, further more preferably 1 minute or shorter.
<42> The method for dyeing hair according to any one of <1> to <41>, comprising, after the hair rinsing step, a drying step of drying the hair, wherein preferably one or more drying treatments selected from the group consisting of towel drying, blow (cold air or hot air) drying, and air drying is performed, and more preferably two or more of the drying treatments are performed in combination.
<43> The method for dyeing hair according to <42>, wherein time from the completion of the rinsing step to the start of the drying step is preferably 20 minutes or shorter, more preferably 17 minutes or shorter, further more preferably 15 minutes or shorter, and a lower limit thereof is not particularly limited and is 10 seconds or longer.

## Examples

Hereinafter, the present invention will be described with reference to Examples. However, the present invention is not limited by the scope of Examples. In the present Examples, various measurements and evaluations were performed by the following methods.

<pH measurement>

pH at 25°C of an A part was measured with a pH meter (F-72, manufactured by HORIBA, Ltd.).

<Color difference ΔE between before and after dyeing>

A hair tress was used. The hair tress was dyed by a dyeing treatment in accordance with a method of each example. The colors of the hair tress before the dyeing treatment and the hair tress after the dyeing treatment were measured at six locations per tress with a colorimeter ("CR-400" manufactured by Konica Minolta, Inc.). In this context,

$L_0^*$, $a_0^*$, and $b_0^*$: the color of the hair tress before the dyeing treatment (average value from measurement at six points)
$L_1^*$, $a_1^*$, and $b_1^*$: the color of the hair tress after the dyeing treatment (average value from measurement at six points)

A color difference ΔE was determined in accordance with the equation given below.

$$\Delta E = \{(\Delta L^*)^2 + (\Delta a^*)^2 + (\Delta b^*)^2\}^{1/2}$$

$\Delta L^*$: $L_1^* - L_0^*$, $\Delta a^*$:$a_1^* - a_0^*$, and $\Delta b^*$: $b_1^* - b_0^*$.

A larger value of ΔE means a higher degree of dyeing and more favorable hair dyeability. It is practically preferred

that ΔE is 20 or more.

<Improvement in hair dyeability>

A difference was determined between ΔE of a hair tress to which a B part mentioned later was not applied and ΔE of a hair tress to which the B part was applied by a method of each example (difference from ΔE without the application of the B part). A larger difference from the ΔE means that hair dyeability was more improved.

<Preparing tress for evaluation>

An untreated hair tress (gray hair tress of a Chinese female having a length of 10 cm and a mass of 1 g, "BM-W-A" manufactured by Beaulax Co., Ltd.,) was cleansed twice with a plain shampoo having the composition described below, blow-dried with hot air of a dryer for 30 minutes, and left to stand overnight or longer at temperature of 25°C under a relative humidity of 65% to prepare a hair tress for evaluation of the degree of dyeing and the improvement in dyeability.

| (Plain shampoo) | (% by mass) |
|---|---|
| ▪ Sodium polyoxyethylene lauryl ether sulf (EMAL E-27C (amount of active ingredateient: 27% by mass), manufactured by Kao Corp.) | 57.4 |
| ▪ Lauramide DEA (AMINON L-02, manufacturedby Kao Corp.) | 1.5 |
| ▪ EDTA-2Na (FROST DS, manufactured by Daii Chemicals Co., Ltd.) | 0.3 |
| ▪ Phosphoric acid (for pH adjustment to 7.0) | |
| ▪ Sodium benzoate | 0.5 |
| ▪ Purified water | balance |
| Total | 100 |

Example 1

The components of each part shown in Table 1 were blended at the blend ratio described in Table 1, and mixed until homogeneous to prepare an A part and a B part.

A hair tress in the state shown in Table 1 was coated with the A part at the bath ratio (dry mass of the hair : mass of the A part) shown in Table 1, and left to stand for the time shown in Table 1 (step 1). The hair tress coated with the A part was coated with the B part at the bath ratio (dry mass of the hair : mass of the component (b)) shown in Table 1, and the B part was rubbed onto the hair tress for spreading for the time shown in Table 1 (step 2). Then, the hair tress was washed with water (approximately 40°C) for 30 seconds (rinsing step). After a lapse of 15 minutes, the hair tress was blow-dried with cold air of a dryer for 15 minutes (drying step). The hair tress thus treated by drying was measured and evaluated by the methods described above. For the evaluation of improvement in hair dyeability, the evaluation was performed by determining a difference from ΔE of Comparative Example 1 mentioned later in which the step of coating the hair tress with the B part was not performed. The results are shown in Table 1.

All the amounts of components blended (% by mass) described in Table 1 are the amount of an active ingredient, and the total of the A part and the B part reaches 100% by mass, respectively.

Comparative Example 1

A hair dyeing treatment, measurements, and evaluations were performed in the same manner as in Example 1 except that the step of coating the hair tress with the B part (step 2) was not performed. The results are shown in Table 1.

Comparative Example 2

A hair dyeing treatment, measurements, and evaluations were performed in the same manner as in Example 1 except that the step of coating the hair tress with the B part (step 2) was performed, and the hair tress was washed with water (approximately 40°C) for 30 seconds (rinsing step), after a lapse of 15 minutes, blow-dried with cold air of a dryer for 15 minutes, and then coated with the A part (step 1). The results are shown in Table 1.

Comparative Example 3

A hair dyeing treatment, measurements, and evaluations were performed in the same manner as in Example 1

except that the hair tress subjected to the step 1, the rinsing step, and the drying step was coated with the B part. The results are shown in Table 1.

Comparative Example 4

The components shown in Table 1 were blended at the blend ratio described in Table 1, and mixed until homogeneous to prepare an A part. A hair tress in the state shown in Table 1 was coated with the A part at the bath ratio (dry mass of the hair : mass of the A part) shown in Table 1, and left to stand for the time shown in Table 1 (step 1). Then, the hair tress was washed with water (approximately 40°C) for 30 seconds (rinsing step). After a lapse of 15 minutes, the hair tress was blow-dried with cold air of a dryer for 15 minutes (drying step). The hair tress thus treated by drying was measured and evaluated by the methods described above. The results are shown in Table 1.

[Table 1]

**Table 1**

| | | Example 1 | Comparative Example 1 | Comparative Example 2 | Comparative Example 3 | Comparative Example 4 |
|---|---|---|---|---|---|---|
| A part | (a1) Mixture of 5,6-dihydroxyindole and 5,6-dihydroxyindole-2-carboxylic acid | 0.25 | 0.25 | 0.25 | 0.25 | 0.25 |
| | (a2) Monoethanolamine | 3 | 3 | 3 | 3 | 3 |
| | (b) $MgCl_2 \cdot 6H_2O$ ($MgCl_2$ concentration) | | | | | 0.403 |
| | 75% phosphoric acid | q.s. | q.s. | q.s. | q.s. | q.s. |
| | Water | Balance | Balance | Balance | Balance | Balance |
| | pH | 10.2 | 10.2 | 10.2 | 10.2 | 10.2 |
| Application conditions of A part | State of hair tress before application of A part | Dry hair | Dry hair | Dry hair | Dry hair | Dry hair |
| | Bath ratio (hair : A part) | 1:3.33 | 1:3.33 | 1:3.33 | 1:3.33 | 1:3.33 |
| | Bath ratio (hair : component (b)) | | | | | 1:0.013 |
| | Time for which sample was left to stand (min) | 10 | 10 | 10 | 10 | 10 |
| B part | (b) $MgCl_2 \cdot 6H_2O$ ($MgCl_2$ concentration) | 2 | | 2 | 2 | |
| | Water | Balance | | Balance | Balance | |
| Application conditions of B part | Bath ratio (hair : component (b)) | 1:0.013 | | 1:0.013 | 1:0.013 | |
| | Spreading time (min) | 1 | | 1 | 1 | |

(continued)

| | | Example 1 | Comparative Example 1 | Comparative Example 2 | Comparative Example 3 | Comparative Example 4 |
|---|---|---|---|---|---|---|
| Order of treatment | | Application of A part ↓ Application of B part ↓ Rinsing ↓ Drying | Application of A part ↓ Rinsing ↓ Drying | Application of B part ↓ Application of A part ↓ Rinsing ↓ Drying | Application of A part ↓ Rinsing ↓ Drying ↓ Application of B part | Mixing of A part with component (b) ↓ Application of A part ↓ Rinsing ↓ Drying |
| Hair dyeability evaluation | Color difference ΔE between before and after dyeing | 46.2 | 40.7 | 38.1 | 43.3 | 36.2 |
| | Difference from ΔE without application of B part | 5.5 | - | -2.5 | 2.7 | -4.5 |

Example 2

The components of each part shown in Table 2 were blended at the blend ratio described in Table 2, and mixed until homogeneous to prepare an A part and a B part.

A hair tress in the state shown in Table 2 was coated with the A part at the bath ratio (dry mass of the hair : mass of the A part) shown in Table 2, and left to stand for the time shown in Table 2 (step 1). The hair tress coated with the A part was coated with the B part at the bath ratio (dry mass of the hair : mass of the component (b)) shown in Table 2, and the B part was rubbed onto the hair tress for spreading for the time shown in Table 2 (step 2). Then, the hair tress was washed with water (approximately 40°C) for 30 seconds (rinsing step). After a lapse of 15 minutes, the hair tress was blow-dried with cold air of a dryer for 15 minutes (drying step). The hair tress thus treated by drying was measured and evaluated by the methods described above. For the evaluation of improvement in hair dyeability, the evaluation was performed by determining a difference from ΔE of Comparative Example 5 mentioned later in which the step of coating the hair tress with the B part was not performed. The results are shown in Table 2.

All the amounts of components blended (% by mass) described in Table 2 are the amount of an active ingredient, and the total of the A part and the B part reaches 100% by mass.

Comparative Example 5

A hair dyeing treatment, measurements, and evaluations were performed in the same manner as in Example 2 except that the step of coating the hair tress with the B part (step 2) was not performed. The results are shown in Table 2.

Comparative Example 6

A hair dyeing treatment, measurements, and evaluations were performed in the same manner as in Example 2 except that the step of coating the hair tress with the B part (step 2) was performed, and the hair tress was washed with water (approximately 40°C) for 30 seconds (rinsing step), after a lapse of 15 minutes, blow-dried with cold air of a dryer for 15 minutes, and then coated with the A part (step 1). The results are shown in Table 2.

Comparative Example 7

A hair dyeing treatment, measurements, and evaluations were performed in the same manner as in Example 2 except that the hair tress subjected to the step 1, the rinsing step, and the drying step was coated with the B part. The results are shown in Table 2.

Comparative Example 8

The components shown in Table 2 were blended at the blend ratio described in Table 2, and mixed until homogeneous to prepare an A part. A hair tress in the state shown in Table 2 was coated with the A part at the bath ratio (dry mass of the hair : mass of the A part) shown in Table 2, and left to stand for the time shown in Table 2 (step 1). Then, the hair

tress was washed with water (approximately 40°C) for 30 seconds (rinsing step). After a lapse of 15 minutes, the hair tress was blow-dried with cold air of a dryer for 15 minutes (drying step). The hair tress thus treated by drying was measured and evaluated by the methods described above. The results are shown in Table 2.

[Table 2]

Table 2

| | | Example 2 | Comparative Example 5 | Comparative Example 6 | Comparative Example 7 | Comparative Example 8 |
|---|---|---|---|---|---|---|
| A part | (a1) Mixture of 5,6-dihydroxyindole and 5,6-dihydroxyindole-2-carboxylic acid | 0.5 | 0.5 | 0.5 | 0.5 | 0.5 |
| | (a2) Monoethanolamine | 3 | 3 | 3 | 3 | 3 |
| | (b) NaCl | | | | | 13 |
| | 75% phosphoric acid | q.s. | q.s. | q.s. | q.s. | q.s. |
| | Water | Balance | Balance | Balance | Balance | Balance |
| | pH | 10.2 | 10.2 | 10.2 | 10.2 | 10.2 |
| Application conditions of A part | State of hair tress before application of A part | Dry hair | Dry hair | Dry hair | Dry hair | Dry hair |
| | Bath ratio (hair : A part) | 1:1 | 1:1 | 1:1 | 1:1 | 1:1 |
| | Bath ratio (hair : component (b)) | | | | | 1:0.13 |
| | Time for which sample was left to stand (min) | 10 | 10 | 10 | 10 | 10 |
| B part | (b) NaCl | 67 | | 67 | 67 | |
| | Sodium laureth sulfate | 8.9 | | 8.9 | 8.9 | |
| | Water | Balance | | Balance | Balance | |
| Application conditions of B part | Bath ratio (hair : component (b)) | 1:0.13 | | 1:0.13 | 1:0.13 | |
| | Spreading time (min) | 1 | | 1 | 1 | |
| Order of treatment | | Application of A part ↓ Application of B part ↓ Rinsing ↓ Drying | Application of A part ↓ Rinsing ↓ Drying | Application of B part ↓ Application of A part ↓ Rinsing ↓Drying | Application of A part ↓ Rinsing ↓ Drying ↓Application of B part | Mixing of A part with component (b) ↓ Application of A part ↓ Rinsing ↓Drying |

(continued)

| | | Example 2 | Comparative Example 5 | Comparative Example 6 | Comparative Example 7 | Comparative Example 8 |
|---|---|---|---|---|---|---|
| Hair dyeability evaluation | Color difference ΔE between before and after dyeing | 46.3 | 44.2 | 43.5 | 44.1 | 34.3 |
| | Difference from ΔE without application of B part | 2.2 | - | -0.7 | -0.02 | -9.9 |

Examples 3 and 4

The components of each part shown in Table 3 were blended at the blend ratio described in Table 3, and mixed until homogeneous to prepare an A part and a B part.

A hair tress in the state shown in Table 3 was coated with the A part at the bath ratio (dry mass of the hair : mass of the A part) shown in Table 3, and left to stand for the time shown in Table 3 (step 1). The hair tress coated with the A part was coated with the B part at the bath ratio (dry mass of the hair : mass of the component (b)) shown in Table 3, and the B part was rubbed onto the hair tress for spreading for the time shown in Table 3 (step 2). Then, the hair tress was washed with water (approximately 40°C) for 30 seconds (rinsing step). Immediately thereafter, the hair tress was blow-dried with cold air of a dryer for 15 minutes (drying step). The hair tress thus treated by drying was measured and evaluated by the methods described above. For the evaluation of improvement in hair dyeability, the evaluation was performed by determining a difference from ΔE of Comparative Example 9 mentioned later in which the step of coating the hair tress with the B part was not performed. The results are shown in Table 3.

All the amounts of components blended (% by mass) described in Table 3 are the amount of an active ingredient, and the total of the A part and the B part reaches 100% by mass.

Comparative Example 9

A hair dyeing treatment, measurements, and evaluations were performed in the same manner as in Examples 3 and 4 except that the step of coating the hair tress with the B part (step 2) was not performed. The results are shown in Table 3.

[Table 3]

Table 3

| | | Example 3 | Example 4 | Comparative Example 9 |
|---|---|---|---|---|
| A part | (a1) Mixture of 5,6-dihydroxyindole and 5,6-dihydroxyindole-2-carboxylic acid | 0.25 | 0.25 | 0.25 |
| | (a2) Monoethanolamine | 3 | 3 | 3 |
| | 75% phosphoric acid | q.s. | q.s. | q.s. |
| | Water | Balance | Balance | Balance |
| | pH | 10.2 | 10.2 | 10.2 |
| Application conditions of A part | State of hair tress before application of A part | Wet hair | Wet hair | Wet hair |
| | Bath ratio (hair : A part) | 1:1 | 1:1 | 1:1 |
| | Time for which sample was left to stand (min) | 10 | 10 | 10 |

(continued)

| | | Example 3 | Example 4 | Comparative Example 9 |
|---|---|---|---|---|
| B part | (b)$(CH_3COO)_2Ca\cdot H_2O$($(CH_3COO)_2Ca$ concentration) | 10 | | |
| | (b) $K_2SO_4$ | | 10 | |
| | Water | Balance | Balance | |
| Application conditions of B part | Bath ratio (hair : component (b)) | 1:0.01 | 1:0.01 | |
| | Spreading time (min) | 1 | 1 | |
| Order of treatment | | Application of A part ↓Application of B part ↓ Rinsing ↓ Drying | Application of A part ↓Application of B part ↓ Rinsing ↓ Drying | Application of A part ↓Rinsing ↓ Drying |
| Hair dyeability evaluation | Color difference ΔE between before and after dyeing | 23.2 | 23.3 | 19.1 |
| | Difference from ΔE without application of B part | 4.1 | 4.2 | - |

Examples 5 to 8

The components of each part shown in Table 4 were blended at the blend ratio described in Table 4, and mixed until homogeneous to prepare an A part and a B part.

A hair tress in the state shown in Table 4 was coated with the A part at the bath ratio (dry mass of the hair : mass of the A part) shown in Table 4, and left to stand for the time shown in Table 4 (step 1). The hair tress coated with the A part was coated with the B part at the bath ratio (dry mass of the hair : mass of the component (b)) shown in Table 4, and the B part was rubbed onto the hair tress for spreading for the time shown in Table 4 (step 2). Then, the hair tress was washed with water (approximately 40°C) for 30 seconds (rinsing step). Immediately thereafter, the hair tress was blow-dried with cold air of a dryer for 15 minutes (drying step). The hair tress thus treated by drying was measured and evaluated by the methods described above. For the evaluation of improvement in hair dyeability, the evaluation was performed by determining a difference from ΔE of Comparative Example 11 mentioned later in which the step of coating the hair tress with the B part was not performed. The results are shown in Table 4.

All the amounts of components blended (% by mass) described in Table 4 are the amount of an active ingredient, and the total of the A part and the B part reaches 100% by mass.

Comparative Example 10

A hair dyeing treatment, measurements, and evaluations were performed in the same manner as in Examples 5 to 8 except that no component (b) was blended into the B part. The results are shown in Table 4.

Comparative Example 11

A hair dyeing treatment, measurements, and evaluations were performed in the same manner as in Examples 5 to 8 except that the step of coating the hair tress with the B part (step 2) was not performed. The results are shown in Table 4.

[Table 4]

Table 4

| | | Example 5 | Example 6 | Example 7 | Example 8 | Comparative Example 10 | Comparative Example 11 |
|---|---|---|---|---|---|---|---|
| A part | (a1) Mixture of 5,6-dihydroxyindole and 5,6-dihydroxyindole-2-carboxylic acid | 0.25 | 0.25 | 0.25 | 0.25 | 0.25 | 0.25 |
| | (a2) Monoethanolamine | 3 | 3 | 3 | 3 | 3 | 3 |
| | 75% phosphoric acid | q.s. | q.s. | q.s. | q.s. | q.s. | q.s. |
| | Water | Balance | Balance | Balance | Balance | Balance | Balance |
| | pH | 10.2 | 10.2 | 10.2 | 10.2 | 10.2 | 10.2 |
| Application conditions of A part | State of hair tress before application of A part | Dry hair | Dry hair | Dry hair | Dry hair | Dry hair | Dry hair |
| | Bath ratio (hair : A part) | 1:1 | 1:1 | 1:1 | 1:1 | 1:1 | 1:1 |
| | Time for which sample was left to stand (min) | 10 | 10 | 10 | 10 | 10 | 10 |
| B part | (b) NaCl | 0.01 | 0.01 | 0.1 | 10 | | |
| | Water | Balance | Balance | Balance | Balance | Balance | |
| Application conditions of B part | Bath ratio (hair : component (b)) | 1:0.0001 | 1:0.001 | 1:0.01 | 1:0.1 | 0 | |
| | Spreading time (min) | 1 | 1 | 1 | 1 | 1 | |
| Order of treatment | | Application of A part↓Application of B part ↓ Rinsing ↓ Drying | Application of A part↓Application of B part ↓ Rinsing ↓ Drying | Application of A part↓Application of B part ↓ Rinsing ↓ Drying | Application of A part↓Application of B part ↓ Rinsing ↓ Drying | Application of A part↓Application of B part ↓ Rinsing ↓ Drying | Application of A part ↓ Rinsing ↓ Drying |

(continued)

| | | Example 5 | Example 6 | Example 7 | Example 8 | Comparative Example 10 | Comparative Example 11 |
|---|---|---|---|---|---|---|---|
| Hair dyeability evaluation | Color difference ΔE between before and after dyeing | 20.1 | 20.4 | 22.2 | 29.2 | 18.2 | 18 |
| | Difference from ΔE without application of B part | 2.1 | 2.4 | 4.2 | 11.2 | 0.2 | - |

The components described in Tables 1 to 4 are as follows.

(a1) 5,6-Dihydroxyindole/5,6-dihydroxyindole-2-carboxylic acid mixture: produced by the method described in JP-B-5570161 for use. The molar ratio between the 5,6-dihydroxyindole and the 5,6-dihydroxyindole-2-carboxylic acid was 90:10 (hereinafter, also referred to as "DHI"). The component (a1) was prepared as a solution containing 1% by mass thereof in $H_2O$/ethanol = 4/1 (wt/wt), which was blended with A part.
(b) NaCl: Sodium chloride (Roasted Salt S: Nippon Salt Manufacturing Co., Ltd.)
(b) $MgCl_2 \cdot 6H_2O$: Magnesium chloride hexahydrate (FUJIFILM Wako Pure Chemical Corp.), adjusted such that the concentration of $MgCl_2$ in the B part was the concentration described in each table
(b) $(CH_3COO)_2Ca \cdot H_2O$: Calcium acetate monohydrate (FUJIFILM Wako Pure Chemical Corp.), adjusted such that the concentration of $(CH_3COO)_2Ca$ in the B part was the concentration described in each table
(b) $K_2SO_4$: Potassium sulfate (FUJIFILM Wako Pure Chemical Corp.)
Sodium laureth sulfate: (27% aqueous solution: manufactured by Kao Corp., EMAL 227-PH11)

As is evident from Tables 1 to 4, the method of the present invention causes no damage ascribable to oxidation, achieves stable hair dyeing performance of a melanin precursor by a simple process, and can further improve hair dyeability.

Formulation Example 1

| | |
|---|---|
| (a1) DHI | 0.5% |
| (a2) Monoethanolamine | 3% |
| Ascorbic acid | 0.6% |
| Sodium sulfite | 0.5% |
| Propylene glycol | 2% |
| QUARTAMIN 86W | 1% |
| NIKKOL BC-2 | 2% |
| NIKKOL BC-40 | 2% |
| KALKOL 8098 | 3% |
| KALKOL 220-80 | 3% |
| 75% phosphoric acid | q.s. |
| Water | Balance |
| pH 10.2 | |

QUARTAMIN 86W: Aqueous solution containing 28% stearyl trimonium chloride (KAO Corp.)
NIKKOL BC-2: Ceteth-2(Nippon Surfactant Industries Co., Ltd.)
NIKKOL BC-40: Ceteth-40(Nippon Surfactant Industries Co., Ltd.)
KALKOL 8098: Stearyl alcohol (KAO Corp.)
KALKOL 220-80: Behenyl alcohol (KAO Corp.)

The components shown in Formulation Example 1 were mixed until homogeneous to prepare an A part. Hair was treated using the A part and a separately prepared B part in the same manner as the method shown in Example 1. As a result, favorable hair dyeability was shown as compared with using no B part.

Formulation Example 2

| | |
|---|---|
| (a1) DHI | 0.075% |
| (a2) Monoethanolamine | 1% |
| Ascorbic acid | 0.4% |
| Sodium sulfite | 0.35% |
| Propylene glycol | 2% |

(continued)

| | |
|---|---|
| QUARTAMIN 86W | 3% |
| NIKKOL BC-2 | 0.5% |
| NIKKOL BC-40 | 1% |
| KALKOL 8098 | 2% |
| KALKOL 220-80 | 4% |
| 75% phosphoric acid | q.s. |
| Water | Balance |
| pH 10.2 | |

QUARTAMIN 86W: Aqueous solution containing 28% stearyl trimonium chloride (KAO Corp.)
NIKKOL BC-2: Ceteth-2(Nippon Surfactant Industries Co., Ltd.)
NIKKOL BC-40: Ceteth-40(Nippon Surfactant Industries Co., Ltd.)
KALKOL 8098: Stearyl alcohol (KAO Corp.)
KALKOL 220-80: Behenyl alcohol (KAO Corp.)

The components shown in Formulation Example 2 were mixed until homogeneous to prepare an A part. Hair was treated with the A part and a separately prepared B part in the same manner as the method shown in Example 2. As a result, favorable hair dyeability was shown as compared with using no B part.

Industrial Applicability

The present invention can provide a method for dyeing hair which causes no damage ascribable to oxidation, achieves stable hair dyeing performance of a melanin precursor by a simple process, and can further improve hair dyeability.

## Claims

**1.** A method for dyeing hair, comprising in order:

step 1: applying an A part comprising the following components (a1) and (a2):

(a1) a compound of the following formula (1) or a salt thereof:

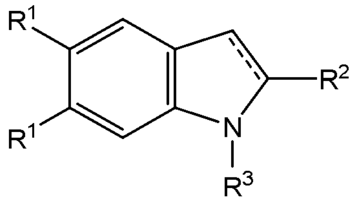

(1)

wherein the broken line represents the presence or absence of a $\pi$ bond; $R^1$ represents a hydroxy group or an acetoxy group; $R^2$ represents a hydrogen atom or -COOR, wherein R represents a hydrogen atom, a methyl group, or an ethyl group; and $R^3$ represents a hydrogen atom, an acetyl group, a methyl group, or an ethyl group, and
(a2) an alkali agent

and having pH of from 8 to 11 to hair; and
step 2: applying a B part comprising the following component (b):
(b) an alkali metal salt or an alkaline earth metal salt selected from the group consisting of an organic acid salt having 6 or less carbon atoms and an inorganic salt to the hair,
the method having no step of rinsing hair between the step 1 and the step 2 and further comprising the step of rinsing hair after the step 2.

**2.** The method for dyeing hair according to claim 1, wherein the component (b) is one or more members selected from the

group consisting of chloride, carboxylate, sulfate, and carbonate of sodium, potassium, calcium, or magnesium.

**3.** The method for dyeing hair according to claim 1 or 2, wherein a content of the component (a1) in the A part is from 0.05 to 1.0% by mass.

**4.** The method for dyeing hair according to any one of claims 1 to 3, wherein in the step 2, the B part is applied to the hair at a bath ratio (dry mass of the hair : mass of the component (b)) of 1:0.0001 or more.

**5.** The method for dyeing hair according to any one of claims 1 to 4, wherein the component (a2) comprises one or more members selected from the group consisting of ammonia and alkanolamine.

**6.** The method for dyeing hair according to any one of claims 1 to 5, wherein neither the A part nor the B part contains an oxidizing agent.

## INTERNATIONAL SEARCH REPORT

International application No.
**PCT/JP2024/002645**

**A. CLASSIFICATION OF SUBJECT MATTER**

***A61K 8/49***(2006.01)i; ***A61K 8/02***(2006.01)i; ***A61K 8/19***(2006.01)i; ***A61K 8/20***(2006.01)i; ***A61K 8/23***(2006.01)i; ***A61K 8/36***(2006.01)i; ***A61K 8/41***(2006.01)i; ***A61Q 5/06***(2006.01)i
FI: A61K8/49; A61K8/02; A61Q5/06; A61K8/36; A61K8/23; A61K8/20; A61K8/19; A61K8/41

According to International Patent Classification (IPC) or to both national classification and IPC

**B. FIELDS SEARCHED**

Minimum documentation searched (classification system followed by classification symbols)

A61K8/49; A61K8/02; A61K8/19; A61K8/20; A61K8/23; A61K8/36; A61K8/41; A61Q5/06

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 5-058860 A (L'OREAL) 09 March 1993 (1993-03-09) claims, paragraphs [0023], [0043]-[0044], examples | 1-6 |
| Y | WO 2021/131868 A1 (KAO CORPORATION) 01 July 2021 (2021-07-01) claims, paragraphs [0008], [0023], [0040]-[0043] | 1-6 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 February 2024** | **12 March 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (July 2022)

**INTERNATIONAL SEARCH REPORT**
**Information on patent family members**

International application No.
**PCT/JP2024/002645**

| Patent document cited in search report | Publication date (day/month/year) | Patent family member(s) | Publication date (day/month/year) |
|---|---|---|---|
| JP 5-058860 A | 09 March 1993 | US 5275626 B1<br>claims, column 5, lines 59-65, column 9, examples<br>EP 498707 A1 | |
| WO 2021/131868 A1 | 01 July 2021 | US 2023/0048023 A1<br>claims, paragraphs [0018], [0067], [0069]-[0076]<br>EP 4082620 A1<br>CN 114828817 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP SHO63170309 A
- JP 63170309 A
- JP HEI1319413 A
- JP 1319413 A
- JP HEI8505155 A
- JP 8605155 A
- JP 5570161 B